(19) Europäisches Patentamt
European Patent Office
Office européen des brevets

(11) **EP 2 525 773 B1**

(12) **EUROPEAN PATENT SPECIFICATION**

(45) Date of publication and mention
of the grant of the patent:
**12.02.2020 Bulletin 2020/07**

(21) Application number: **11769500.7**

(22) Date of filing: **13.04.2011**

(51) Int Cl.:
*A61K 8/37* *(2006.01)*          *A61K 8/30* *(2006.01)*
*A61K 8/25* *(2006.01)*          *A61K 8/19* *(2006.01)*
*A61K 9/06* *(2006.01)*          *A61Q 3/02* *(2006.01)*
*A61Q 3/00* *(2006.01)*          *A61K 8/81* *(2006.01)*
*A61K 8/87* *(2006.01)*          *A61K 8/26* *(2006.01)*

(86) International application number:
**PCT/US2011/032245**

(87) International publication number:
**WO 2011/130362 (20.10.2011 Gazette 2011/42)**

(54) **EASILY APPLIABLE, STORAGE STABLE, RADIATION-CURABLE, PIGMENTED, ARTIFICIAL NAIL GEL COATINGS**

LEICHT AUFBRINGBARE, LAGERSTABILE, STRAHLUNGSHÄRTBARE UND PIGMENTIERTE KÜNSTLICHE NAGELGELBESCHICHTUNGEN

REVÊTEMENTS DE GEL DE CLOU ARTIFICIELS, PIGMENTÉS, RÉTICULABLES PAR RADIATION, STABLES AU STOCKAGE ET FACILES À APPLIQUER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.04.2010 US 798953**
**16.07.2010 US 804253**

(43) Date of publication of application:
**28.11.2012 Bulletin 2012/48**

(73) Proprietor: **Mycone Dental Supply Co. Inc.**
**Chery Hill, NJ 08002 (US)**

(72) Inventors:
• **KOZACHECK, Joseph, E.**
**Newton Square**
**PA 19073 (US)**
• **LEIN, George, M.**
**Elkton, MD 21912 (US)**

(74) Representative: **Lavoix**
**Bayerstrasse 83**
**80335 München (DE)**

(56) References cited:
**GB-A- 2 452 566**       **US-A- 3 899 611**
**US-A- 4 929 403**       **US-A- 5 407 666**
**US-A- 5 407 666**       **US-A- 5 985 951**
**US-A1- 2007 207 096**   **US-B1- 6 244 274**
**US-B1- 6 244 274**       **US-B2- 6 599 958**
**US-B2- 6 599 958**

Remarks:
The file contains technical information submitted after the application was filed and not included in this specification

**Description**

**CROSS-REFRENCE TO RELATED APPLICATIONS**

Benefit of U.S. patent applications Ser. No. 12/804,253, filed July 16, 2010 and Ser. No. 12/798,953, filed April 14, 2010 is claimed.

**BACKGROUND OF THE INVENTION**

This invention relates to the field of radiation-curable gels useful for cosmetic adornment of natural nails, artificial fingernails, toenails and artificial nail extensions.

The use of radiation-curable gels in formation of nail enhancements or artificial nails has been an important part of the cosmetic industry since it was first introduced. US Pat. 4,682,612, describing the use of actinic radiation-curable compositions suitable for preparation of artificial nails, is representative of this technology.

Ultra-violet radiation (UV) is the most conventional form of radiation used to cure gels in this art, however, visible light curing systems are also known. Professional nail technicians most typically apply UV curable gels designed for sculpting nails. Such UV-curable gels are usually composed of acrylic or methacrylic monomers and oligomers in a gel-like state that requires curing under a UV lamp. Such nail finishes can be applied directly to natural fingernails or toenails, or alternatively can be applied to nail extensions bonded to fingernails. In many cases, the artificial nails are coated with conventional nail polish after they are cured.

In order to avoid the need to coat the artificial nails or natural nails with conventional nail polish containing high levels of solvent, in more recent years the preparation of gels containing colorants, particularly pigments, has become known in this art.

Present color pigmented gel formulations are highly viscous and are not as easy to apply as per the ease of applying nail polish for example. Lower viscosity gels are preferred since their application properties are similar to standard nail polishes. However, the use of lower viscosity gels, leads to colored gel formulations having a limited shelf life due to pigment settling and the formation of hard packs. Once the hard pack is formed, it is difficult or nearly impossible to resuspend the pigment into the gel as a homogeneous composition.

Sculpting, or builder gels, are very difficult to apply due to their high viscosity and they often need to be filed down and reapplied multiple times to build up a nail. Low viscosity UV curable gels, as per the composition of the invention stated herein, can be applied as a thin coat and are typically applied as a single coating. A second coating is optional. Therefore, there is an unmet need in the art for a low viscosity UV curable nail gel coating with good control of flow when applied to nails in combination with a homogeneous distribution of color pigment that does not easily form hard packs upon storage. Storage shelf life at ambient conditions is critical for a commercially acceptable product.

Prior references demonstrate the addition of agents to modify the rheological properties of solvent based nail polishes and high viscosity UV sculpting gels, but say nothing in regards to an agent that will impart a reduction in high shear viscosity and impart stability to pigmented low viscosity UV curable gel coatings. US 6244274 for "Thixotropic Polymerizable Nail Sculpting Compositions" (Sirdesai et. al.) claims a single composition and method to modify the rheological properties of a reactive UV curable sculpting gel. While rheological modification of a builder gel may make it easier to apply, the viscosity at application of this type of gel is necessarily much higher than that of a coating gel in order to allow for the building of the artcificial nail. Sirdesai et al discloses a sculpting high viscosity UV curable gel containing low or no pigments. The levels exemplified in Sirdesai et al are typical of those used to provide a natural look. In addition, color pigments are not exemplified and there is no mention of pigment stability upon storage.

Current UV curable gels containing pigments are of high viscosity making them difficult to apply and the current state of the art UV curable gels of lower viscosity have been either without pigment or contain only very low levels of pigment.

Although, US 6,555,096, "Nail Enamel Composition Containing a Urea-modified Thixotropic Agent in a Solvent System" (Carrion et al) claims a non-UV curable nail enamel and a urea modified thixotropic agent in a solvent and mentions in the description that the thixotropic agent acts to suspend the colorant, the described formulation is not a UV curable gel. US 4,222,908, "Thixotropic Nail Enamel" by Ikeda et. al. also utilizes a gelling agent (organically modified montmorillonite clay) in a non-UV curable thixotropic nail enamel. Furthermore, Ikeda describes the use of such gelling agents as a way to prevent separation of pigments and pearl essences.

It should also be stated here that large quantities of organic solvents, which are undesirable in UV curable gels, are used in the relatively low viscosity nail enamels. For example US 5,985,951 (Cook) describes a UV curable coating which needs about 65-75 wt % suitable solvent to solubilize the "solvent based modified cellulose ester coating composition." This is an example of typical amounts of unreactive solvent in commercial nail coatings. According to Cook, the solvent, in addition to the coating, is applied to nails and emits significant amounts of unpleasant and potentially harmful solvent vapors.

In US patent 6,051,242, Patel describes a quick drying nail polish coating composition containing a thixotropic additive which is comprised of two different solvent dissolved polymer systems for primary and secondary film formation. The nail polish composition is comprised of solvent levels from 8 to 80 wt percent, preferably 45 - 55 % solvent. The nail polish material also only contains about 1% to 20% reactive species selected from a specific group of monomers, oligomers, and polymers compatible with the primary and secondary film-forming polymers. The Patel composition is a typical low viscosity nail polish, with a viscosity of no more than 0,48 Pa.s (4.8 Poise) at low shear rates, and it is not UV cured. The Patel composition also contains a thixotropic agent, but the purpose of the thixotropic agent is to gel the base composition or lacquer.

Patel, et al., U.S. Pat. 5,407,666, disclosed an easily removable, hard, durable nail coating composition comprising 5-65% by weight of an acrylic or cellulosic film-forming polymer, 2-20% by weight photoreactive monomer, 0-10% by weight of a photoreactive substance selected from epoxyacrylate oligomer, urethane acrylate oligomer, and vinyl ether, 0-10% by weight of a thickening agent selected from fumed silica, clay, and pulverized glass, optional (0-5%) by weight pigment, and 2-20% by weight photoinitiator. None of Patel's five formulation examples comprised an oligomer or an organic modified clay.

In addition, a considerable advantage of the use of the UV nail gel for the customer and the person performing the application is the reduced time needed to harden. A customer can spend up to an hour waiting for the solvent in nail enamel to evaporate, while the gel is set in 3 minutes or less.

It is an object of the present invention to provide a UV curable nail coating composition which is wear resistant, easily and quickly applied and cured, has good high and low shear viscosity properties to allow easy and efficient application by professional and non-professional applicators, and has good appearance.

## SUMMARY OF THE INVENTION

These objects, and others which will become apparent from the following disclosure, are achieved by the present invention which comprises in one aspect a composition comprising:

(A) at least 35% by weight polyfunctional polyurethane (meth)acrylate oligomer;
(B) at least 0.1 % by weight pigment;
(C) 0.5% to 10% by weight thixotropic additive selected from the group consisting of organically-modified clay, organically-modified mixed mineral clay, and a mixture thereof;
(D) 0 to about 20% by weight non-reactive solvent;
(E) 0 to no more than 20% by weight film-forming polymer;
(F) 0.1 to 5% by weight photoinitiator;
(G) 0 to 5% by weight dispersant, and
(H) the balance to make 100% by weight of one or more mono-, di-tri-, and tetrafunctional acrylic or methacrylic monomers;

wherein the ratio of oligomer (A) to film forming polymer (E) is greater than 2.5;
and wherein the composition is a UV curable gel suitable for coating natural and artificial nails and forming a wear-resistant adornment, and
wherein the composition has a low shear viscosity at 2/sec shear rate at 25°C of greater than 1.2 Pa.s and a high shear viscosity at 70/sec shear rate at 25°C of less than 2 Pa.s.

In another aspect the invention comprises a method of coating human and artificial nails comprising applying such a composition and curing under actinic radiation such as UV.

The invention also comprises a cured coating resulting from exposure of the composition to actinic radiation.

The composition has a low shear viscosity at 25 C. of greater than 1,2 Pa.s (12 poise) and/or a high shear viscosity at 25 C. of less than 2 Pa.s 20 poise).

The thixotropic additive (C) is selected from the group consisting organically modified clay or organically modified mixed mineral clay, and mixtures thereof. Preferred amounts of thixotropic additive (C) in the composition are up to 10 % by weight, preferably up to about 5% by weight.

In some embodiments there is no film-forming polymer, (E), and no non-reactive solvent, (D) in the composition.

According to a disclosure, the oligomer (A) is present in amounts of at least 35% by weight. Preferred amounts of oligomer are at least about 45%, and more preferably at least 50% by weight. One advantage of use of such large amount of oligomer is the significantly improved wear characteristics such as durability and resistance to chipping properties of the radiation cured coatings. However one disadvantage, in some embodiments, is the inability to remove the coatings with conventional nail polish removers. In such embodiments, abrasion in combination with such conventional nail polish remover is needed.

The problems of limited shelf life due to pigment settling and the formation of hard packs in low viscosity, low VOC, UV curable gels are solved by the present invention which comprises in one aspect a composition comprised of a low viscosity radiation curable color gel coating comprised of a formulation containing the addition of thixotropic additive(s) and in some cases dispersants for additional dispersion stability. The thixotropic color pigmented UV curable gel has prolonged shelf life and long time storage at ambient conditions. In another embodiment, dispersants can be added to improve both the shelf life and to allow for resuspension of pigment to further improve shelf life. In addition, this invention resolves an additional long time problem with its ease of application as it behaves similarly to a commercial nail polish in terms of application to nails but without significant amounts of unpleasant and potentially harmful solvent vapors. Sculpting or builder gels need to be applied by a highly trained professional; the invention herein can be applied by a novice.

In yet another aspect, the invention claims the use of such a highly shelf stable colored UV-curable artificial nail gel.

**DETAILED DESCRIPTION**

According to the invention herein, shelf stable, pigment containing, low viscosity UV curable nail gel coatings can be successfully prepared by utilizing thixotropic additive(s) to prepare such gels. In another embodiment, dispersants can be added to improve both the shelf life and to allow for resuspension of pigment to further improve shelf life at ambient conditions. The thixotropic additive builds in a reduction of high shear viscosity allowing the application of a uniform coating without adversely affecting the rheological properties of the gel. This allows the nail gel to be easily applied to nails at a lower viscosity due to shear thinning properties.

For good shelf stability at ambient conditions (about room temperature or around 25 degrees Celsius) the viscosity of the system at rest should be sufficient to keep pigments suspended as defined by Stoke's law below.

$$V_s = \frac{2}{9} \frac{(\rho_p - \rho_f)}{\mu} g \, R^2$$

wherein:

$V_s$ is the particles' settling velocity (m/s) (vertically downwards if $\rho_p > \rho_f$, upwards if $\rho_p < \rho_t$),
$g$ is the gravitational acceleration (m/s²),
$\rho_p$ is the mass density of the particles (kg/m³),
$\rho_f$ is the mass density of the fluid (kg/m3),
$\mu$ is the fluid's viscosity (in [kg m-1 s-1]), and
R is the radius of the spherical object (in m).

Shear rates under these "system at rest" conditions are in the $10^{-4}$/sec to $10^{-6}$/sec range (as stated in the Rheology Modifiers handbook, David B Braun and Meyer R. Rosen, William Andrew Publishing, 1999, Pg 17). Thus, the thixotropic additive(s) must impart sufficient viscosity under low shear rate conditions to prevent pigment settling and show viscosity reduction upon the applied shear such that good application properties are obtained. Ease of application requires a viscosity under a shear rate of 70/sec of less than 4 Pa.s (40 poise) with less than 3 Pa.s (30 poise) being preferred and less than 2 Pa.s (20 poise) being most preferred. The shear rate for application is generally in the range of 50/sec - 1000/sec.

The UV-curable nail gels can be comprised of no more than about 20% by weight film-forming polymer (E), , however, for optimum wear properties the ratio of oligomer to film forming polymer must be greater than 2.5. Most preferably <5% film-forming polymer is included in the composition. Prior fingernail coating compositions such as those exemplified by Patel,et al. U.S. Pat. 5,407,666, comprise 5 to 65% by weight of film-forming polymers, and low levels, only 5-10% by weight, of oligomer. By "film-forming polymer" we mean those in the prior art such as cellulose polymers and acrylic polymers such as polylmethyl methacrylate, polybutyl methacrylate, polymethyl methacrylate-co-poly ethyl or polybutyl methacrylate, and the like. Other film forming polymers may also be used.

The UV-curable compositions of the invention comprise a significant amount of ethylenically unsaturated monomer (H), in some embodiments about 45 to 65% by weight of the composition, and can have between one and five reactive double bonds. Typical examples include esters and amides of acrylic and methacrylic acid. The esters of acrylic and methacrylic acid are herein termed (meth)acrylic ester. Specific but not limiting examples of mono methyl (meth)acrylic esters include: methyl (meth)acrylate, ethyl (meth)acrylate hydroxypropyl (meth)acrylate (HPMA), ethyl (meth)acrylate, butyl (meth)acrylate, hydroxy ethyl (meth)acrylate (HEMA), butoxyethyl (meth)acrylate, diethylaminoethyl (meth)acrylate, 2-ethylhexyl (meth)acrylate, ethoxyethyl (meth)acrylate, t-butyl aminoethyl (meth)acrylate, methoxyethylene glycol (meth)acrylate, phosphoethyl (meth)acrylate, methoxy propyl (meth)acrylate, methoxy polyethylene glycol(meth)acrylate, phenoxyethylene glycol (meth)acrylate, phenoxypolyethylene glycol (meth)acrylate, 2-hydroxy-3-phenoxypropyl (meth)acrylate, 2-(meth)acryloxyethylsuccinic acid, 2-(meth)acryloylethylphthalic acid, 2-(meth)acryloyloxypropylphthalic acid, stearyl (meth)acrylate, isobornyl (meth)acrylate, 3-chloro-2-hydroxypropyl (meth)acrylate, tetrahydrofurfuryl (meth)acrylate, (meth)acrylamides and allyl monomers. Specific but not limiting examples of difunctional methacryl esters include: 1,4-butane diol di(meth)acrylate, 1,6-hexanediol di(meth)acrylate, 1,9-nonanediol di(meth)acrylate, 1,10-decanediol di(meth)acrylate, neopentyl glycol di(meth)acrylate, 2-methyl-1,8-octane diol di(meth)acrylate, glycerol di(meth)acrylate, ethylene glycol di(meth)acrylate, triethylene glycol di(meth)acrylate, polyethylene glycol di(meth)acrylate, polypropylene glycol di(meth)acrylate, ethoxylated propylene glycol di(meth)acrylate, ethoxylated polypropylene glycol di(meth)acrylate, polyethoxypropoxy di(meth)acrylate, ethoxylated bisphenol A di(meth)acrylate, propoxylated bisphenol A di(meth)acrylate, propoxylated ethoxylated bisphenol A di(meth)acrylate, bisphenol-A glycidyl methacrylate, tricyclodecanedimethanol di(meth)acrylates glycerin di(meth)acrylate, ethoxylated glycerin di(meth)acrylate, bis acrylamides, bis allyl ethers and allyl (meth)acrylates. Examples of tri and or higher (meth)acryloyl esters include trimethylol propane tri(meth)acrylate, ethoxylated glycerin tri(meth)acrylate, ethoxylated trimethylolpropane tri(meth)acrylate, ditrimethylol propane tetra(meth)acrylate, pentaerythritol tri(meth)acrylate, pentaerythritol tetra(meth)acrylate, propoxylated pentaerythritol tetra(meth)acrylate, ethoxylated pentaerythritol tetra(meth)acrylate, dipentaerythritol penta(meth)acrylate, dipentaerythritol hexa(meth)acrylate, and ethoxlated iscyanuric acid tri(meth)acrylates.

In addition to the above-described (meth)acrylate-based polymerizable monomers, other polymerizable monomers, oligomers or polymers of monomers which contain at least one free radical polymerizable group in the molecule may be used without any limitations in the curable gel. These monomers may contain other groups such as carboxyl groups to improve adhesion.

A compound having at least one free radical polymerizable group includes not only a single component but also a mixture of polymerizable monomers. Thus, combinations of two or more materials containing free radical polymerizable groups may be used in combination.

The oligomer component (A) of the compositions of the invention can be, for example, urethane(meth)acrylates, which have at least two or more acryl or methacryl groups and a urethane group. Examples include: urethanes based on aliphatic, aromatic, polyester, and polyether polyols and aliphatic, aromatic, polyester, and polyether diisocyanates capped with (meth)acrylate end-groups. Isocyanate prepolymers can also be used in place of the polyol-diisocyanate core. Other oligomers useful in the present invention include epoxy (meth)acrylates and epoxy urethane (meth)acrylates, having at least two or more acryl or methacryl groups and, optionally, a urethane group. Examples include epoxy (meth)acrylates based on aliphatic or aromatic epoxy prepolymers capped with (meth)acrylate end-groups. A aliphatic or aromatic urethane spacer can be optionally inserted between the epoxy and the (meth)acrylate endgroup(s). Acrylated polyester oligomers, useful in the present invention, have at least two or more acryl or methacryl groups and a polyester core. Acrylated polyether oligomers, useful in the present invention, have at least two or more acryl or methacryl groups and a polyether core. Acrylated acrylate oligomers, useful in the present invention, have at least two or more acryl or methacryl groups and a polyacrylic core. These reactive urethanes, epoxies, polyesters, polyethers and acrylics are available from several suppliers including BASF Corporation, Bayer MaterialScience, Bomar Specialties Co, Cognis Corporation, Cytec Industries Inc, DSM NeoResins, Eternal Chemical Co, Ltd, IGM Resins, Rahn AG, Sartomer USA, LLC, and SI Group, Inc.

The gel compositions of the invention comprise 0.1% by weight, up to about 5% by weight, of a photoinitiator (F). Examples of these include: benzyl ketones, monomeric hydroxyl ketones, polymeric hydroxyl ketones, alpha -amino ketones, acyl phosphine oxides, metallocenes, benzophenone, benzophenone derivatives, and the like. Specific examples include: 1-hydroxy-cyclohexylphenylketone, benzophenone, 2-benzyl-2-(dimethylamino)-1-(4-(4-morpholinyl)phenyl)-1-butanone, 2-methyl-1-(4-methylthio)phenyl-2-(4-morpholinyl)-1-propanone, diphenyl-(2,4,6-trimethylbenzoyl) phosphine oxide, phenyl bis(2,4,6-trimethylbenzoyl) phosphine oxide, benzyl-dimethylketal, isopropylthioxanthone, and mixtures thereof.

Photo accelerators such as aliphatic or aromatic amines may also be included in the gel as well as fillers, inhibitors, plasticizers, polymers, and adhesion promoters.

Suitable pigments (B) which can be incorporated into the color concentrates include barium, calcium and aluminum lakes, iron oxides, chromates, molybdates, cadmiums, metallic or mixed metallic oxides, talcs, carmine, titanium dioxide, chromium hydroxides, ferric ferrocyanide, ultramarines, titanium dioxide coated mica platelets, and/or bismuth oxychlorides. Preferred pigments include D&C Black No. 2, D&C Black No. 3, FD&C Blue No. 1, D&C Blue No. 4, D&C Brown No. 1, FD&C Green No. 3, D&C Green No. 5, D&C Green No. 6, D&C Green No. 8, D&C Orange No. 4, D&C Orange No. 5, D&C Orange No. 10, D&C Orange No. 11, FD&C Red No. 4., D&C Red No. 6, D&C Red No. 7, D&C Red No. 17, D&C Red No. 21, D&C Red No. 22, D&C Red No. 27, D&C Red No. 28, D&C Red No. 30. D&C Red No. 31, D&C Red No. 33, D&C Red No. 34, D&C Red No. 36, FD&C Red No. 40, D&C Violet No. 2, Ext. D&C Violet No. 2, FD&C Yellow No. 5, FD&C Yellow No. 6, D&C Yellow No. 7, Ext. D&C Yellow No. 7, D&C Yellow No. 8, D&C Yellow No. 10, D&C Yellow No. 11, as well as others listed on the FDA color additives website, and Annex IV of the Cosmetic Directive 76/768/EEC, Coloring Agents Permitted in Cosmetics as of March 1, 2010.

Pigment levels in the composition can be from greater than 0.1-wt % up to as much as 20-wt %. Colored pigments are preferred from 0.5 up to 10-wt%. Mixtures of TiO2 and colored pigments are most preferred.

A thixotropic additive is defined herein as an additive that when mixed with a relatively low viscosity gel imparts shelf stability to the pigmented gel wherein the pigment does not readily fall out of the gel to form a hard pack. A hard pack is essentially the material (mainly pigment) that settles and can not be easily redispersed by low shear methods such as shaking or rolling. In most cases the hard pack (pigments) cannot be re-incorporated back into the nail gel. These dry pigment agglomerates, if they are resuspended into the gels and applied with the coating onto the nails, tend to form surface defects in the nail gel coating and poor appearance. In the invention herein, when particles do settle over time in the gel, they do not form a hardpack and they can be easily resuspended with gentle mixing such as shaking and rolling. The thixotropic additive preferably imparts shear thinning properties such that a viscosity reduction of at least a factor of 1.5 occurs over the range of shear from that when the sample is at rest to that encountered under use conditions, e.g., $10^{-6}$/sec to 70/sec, i.e.- the gel is thick (viscous) under normal storage conditions, but flows (becomes thin, less viscous) when stressed such as applying the gel to nails. The thixotropic additive changes the rheological properties of the gel. The thixotropic additive also imparts pseudoplasticity to the system. A thixotropic fluid displays a decrease in viscosity over time at a constant shear rate, while a pseudoplastic fluid displays decreasing viscosity with increasing shear rate. We mention the thixotropic nature of the nail gels here in this application, but one can also state that the nail gels with the thixotropic additive are exhibiting non-Newtonian pseudoplastic behavior.

Thixotropic additives (C) useful in this invention include inorganic and organic based materials, however organic and organic modified inorganic thixotropic additives are highly preferred. Compositions containing organic modified inorganic based thixotropic additives demonstrate higher yield stress relative to viscosity obtained at applied shear rates compared to compositions containing organic thixotropic additives. In practical terms, this is typified by a steeper reduction in viscosity upon adding shear to the system. This allows good fluidity and handling even at low shear rates such as 2/sec.

Examples of inorganic thixotropic additives useful in the invention include but are not limited to: silica, fumed silica such as that available as Aerosil ® from Evonik Industries or Cab-O-Sil® available from Cabot Corporation, diatomaceous earth, bentonite clay, kaolinite, pyrophyllite, sericite, saponite, smectic/vermiculites (montmorillinite, beidillite, nontronite, hectorite, talc, mica, zirconium oxide, zinc oxide, magnesium oxide, and saponite).

The preferred organic modified inorganic thixotropic additives include, for example, calcium stearate, zinc stearate, aluminum stearate, and organic modified clays, including but not limited to: stearalkonium or distearalkonium bentonite and hectorite and others that are available from Elementis Specialties under the trade name of Bentone®. Highly preferred are organic modified mixed mineral thixotropic additives, for example Garamite® additives from Rockwood Additives such as Garamite® 1958.

Examples of organic thixotropic additives include but are not limited to: hydrogenated castor oils, hydrogenated castor oil waxes, inorganically modified castor oils, organically modified castor oils such as those sold by Elementis Specialties under the Thixcin® trademark, triglycerides such as glyceryl tri-12-hydroxy stearate, polyamides and modified polyamides such as 12-hydroxystearic acid diamide of ethylene diamine, 12-hydroxystearicacid diglycolamide, N-stearyl ricinoleamide, N-stearyl stearamide and other polyamide waxes. Included in these polyamide materials are those sold commercially by Kusumoto Chemicals Industries under the Disparlon® trademark, by Lehmann and Voss under the Luvotix® trademark, by Elementis Specialties under the Thixatrol® trademark, polyethylene oxide waxes, urea urethanes believed to be exemplified by those sold by Byk Incorporated as, for example, by Byk-410, Byk-411, and Byk-420, acrylic resins, amine salts of polymeric polyesters, salts of linear polyaminoamide and polymeric polyester, amide solutions of polycarboxylic acid, alkyl sulfonate, alkylallyl sulfonate, colloidal ester, polyester resin such as those sold by Elementis Specialties under the Thixatrol® trademark, phenol resin, melamine resin, epoxy resin, urethane resin, styrene butadiene polymers, polyimide resin, and polyester amides. Materials such as those sold by Byk under the trademarks of Anti-Terra® and Bykumen® can also be used.

Thixotropic additives (C) can be used at amounts of at least 0.5 wt.% and usually not more than 10 wt.%, preferably not more than 5 wt. % and more preferably not more than about 3 wt.% of the gel composition. It is preferred to use quantities from 0.5 to 5.0 wt.% and more preferred to use amounts of 0.5 to 3.0 wt %.

The composition in some embodiments includes a dispersant additive (G) which can be a non-surface active polymer or a surface-active substance added to a gel suspension to improve the separation of particles and to prevent agglomeration. Dispersants consist normally of one or more surfactants or polymers.

Examples of suitable dispersants (G) include but are not limited to: dispersants sold by Buckman Laboratories under the Busperse® trademark, dispersants sold by Byk under the Disperbyk® trademark, dispersants sold by Lubrizol under the Solsperse® trademark, dispersants sold by BASF under the EFKA® trademark, dispersants sold by Kyoeisha Chemcial Co. under the Flowlen® trademark, dipersants sold by Ajinomoto under the Ajisper® trademark, dispersants sold by Cognis under the Texaphor® trademark, dispersants sold by Cytec Industries under the Aerosol® trademark, dispersants sold by Ethox chemicals, dispersants sold by San Nopko, dispersants sold by Kusumoto Chemical under the Disparlon® trademark and dipersants sold by Evonik under the Tego® trademark.

By the term "gel," we mean a radiation-curable composition comprising photoinitiator, ethylenically unsaturated monomers and oligomers wherein the oligomer content is at least about 35% of the composition, having a viscosity suitable for coating natural or artificial nails, or artificial nails and extensions, as well as adorning such nails.

Unreactive solvent (D) is defined herein as a volatile species (such as a low boiling temperature liquid) that evaporates from typical nail enamel formulations and in final UV cured formulations and serves no reactive function in the formulation per se. Although up to about 20% by weight of non-reactive solvent can be used in some embodiments, preferably less than about 10%, more preferably below 8%, and most preferably 0% non-reactive solvent is used in the formulations of the invention. In contrast, examples of reactive solvent are reactive monomers and/or other reactive species remaining in the formulation. The formulations of the invention are comprised of predominantly reactive monomers and or oligomers and mixtures thereof, and these are utilized in amounts of > 60 wt% of the gel formulation, which upon UV activation are the primary components of the resulting UV cured nail gel. The reactive monomers and oligomers can act as "reactive solvents" as they can solubilize different components in the composition.

In the below examples, which further define the embodiments, the gel viscosities are measured at 25 °C, 2/sec shear and 70/sec shear, on a TA Instruments AR500 Rheometer. 2/sec represents a low shear rate and 70/sec represents a high shear rate. In terms of stability of the UV gel, we define syneresis as separation of the pigment and the gel to leave a clear layer at the top of the gel. Ultimately, in some cases a compacted solid hard pack is formed.

## EXAMPLES

### Example 1 (outside claim 1)

To 125 g of a UV-curable gel comprising 58% by weight of an aliphatic polyester based urethane multimethacrylate oligomer, 20% by weight hydroxyethylmethacrylate monomer (HEMA), 20 % by weight hydroxypropyl methacrylate (HPMA) monomer, and 2% of a mixture of Irgacure 184 and Darocure TPO, both available from BASF Resins, as photoinititator was added 25 g of a dispersion which had been prepared by shearing at 10000 rpm (using a Biohomogenizer available from Biospec Products) comprising 14 grams of Aerosil® 300 silica as thixotropic agent (available from Evonik Degussa GmbH) into 686 g of the same UV curable gel described above. To 44.7 g of the resulting dispersion was added 5.25 g of a 24% wt/wt dispersion of FD&C Red #7 Calcium Lake pigment in a polyether diacrylate monomer. The resulting composition had a viscosity at 25 °C of 1,8 Pa.s (18 poise) at 2/sec shear rate, and a high shear viscosity of 1,3 Pa.s (13 poise) at 70/sec shear rate. The mixture was allowed to stand at ambient temperature and periodically checked for settling. After 26 days, no settling had occurred.

### Example 1A (Comparative)

To 44.7 g of the UV curable gel used in Example 1 (to which no silica thixotropic agent had been added) was added 5.25 g of the Red #7 Calcium Lake dispersion used in Example 1. After standing 5 days, a clear pigment free layer had formed on the top of the sample.

### Example 2 (outside claim 1)

To 125 g of a UV-curable gel comprising 58% by weight of an aliphatic polyester-based urethane multimethacrylate oligomer, 20% by weight hydroxyethylmethacrylate (HEMA), 20 % by weight hydroxypropyl methacrylate (HPMA) and 2% of a mixture of Irgacure 184 and Darocure TPO, both available from BASF Resins, as photoinititator was added 25 g of a dispersion containing the thixotropic additive (Aerosil® 300 silica). The dispersion had been prepared by shearing at 10000 rpm (using a Biohomogenizer available from Biospec Products) 14 grams of Aerosil® 300 (available from Evonik Degussa GmbH) into 686 g of the same UV curable gel described above. To 47.7 g of the resulting dispersion was added 2.3 grams of a 58% wt/wt dispersion of titanium dioxide pigment in a diacrylate monomer. The resulting composition had a viscosity at 25 °C of 2 Pa.s (20 poise) at 2/sec shear rate and 1,6 Pa.s (16 poise) at 70/sec shear rate. The mixture was allowed to stand at ambient temperature and periodically checked for settling. After 26 days, no settling had occurred.

### Example 2A (Comparative)

To 47.7 g of the UV curable gel used in Example 2, to which no silica dispersion had been added, was added 2.3 g of the titanium dioxide dispersion used in Example 1. After standing 5 days, nearly all pigment had settled.

### Example 3 of the invention

To 122 g of a UV-curable gel comprising 58% by weight an aliphatic polyester based urethane multimethacrylate oligomer, 20% by weight hydroxyethylmethacrylate, 20% by weight hydroxypropyl methacrylate and 2% of a mixture of Irgacure 184 and Darocure TPO, both available from BASF Resins,as photoinititator was added 28 g of a dispersion which had been prepared by shearing: 32 g of Garamite® 1958, available from Rockwood Specialties, and 368 g of the same UV curable gel as described above in a Cowles laboratory mixer at 1500 rpm. To 44.7 g of the resulting dispersion was added 5.3 g of a 24% wt/wt dispersion of FD&C Red #7 Calcium Lake Pigment dispersed in a diacrylate monomer. The resulting composition had a viscosity at 25 °C of 2,1 Pa.s (21 poise) at 2/sec shear rate and 1,6 Pa.s (16 poise) at 70/sec shear rate. The mixture was allowed to stand at ambient temperature and periodically checked for settling. After 26 days, no settling had occurred.

### Example 4 of the invention

To 450 g of a UV-curable gel comprised of 58% by weight of an aliphatic polyester based urethane multimethacrylate oligomer, 20% by weight hydroxyethylmethacrylate (HEMA), 20% by weight hydroxypropyl methacrylate (HPMA) and 2% of a mixture of Irgacure 184 and Darocure TPO, both available from BASF Resins, as photoinitiator was added 150 g of a dispersion which was prepared by shearing 40 g of a bentonite clay Garamite 1958 available from Rockwood Specialties, and 460 g of the same UV curable gel as described above in a Cowles laboratory mixer at 1500 rpms. To 45.2 g of the resulting dispersion was added 4.8 g of Red #7 LT Paste concentrate available from Tevco, Inc, which is Red #7 Lake Pigment dispersed in a mixture of ethyl acetate, butyl acetate, isopropyl alcohol, nitrocellulose and Red 7 lake. The resulting composition had a viscosity at 25 °C of 1,2 Pa.s (12 poise) at 2/sec shear rate and 0,79 Pa.s (7.9 poise) at 70/sec shear rate. The mixture was subdivided and one sample was maintained in a 50 °C oven and the other at ambient temperature and periodically checked for settling. After 34 days, neither sample showed signs of settling.

### Example 5 of the invention

To 406.2 g of a UV-curable gel comprised of 58% by weight of an aliphatic polyester based urethane multimethacrylate oligomer, 20% by weight hydroxyethylmethacrylate, 20% by weight hydroxypropyl methacrylate and 2% of a mixture of Irgacure 184 and Darocure TPO, both available from BASF Resins, as photoinitiator was added 94 g of a dispersion which was prepared by shearing 40 g of a bentonite clay Garamite 1958 available from Rockwood Specialties, and 460 g of the same UV curable gel as described above in a Cowles laboratory mixer at 1500 rpms. To 95.2 g of the resulting dispersion was added 6.8% of a color mixture consisting of 0.39% Yellow #5 Paste, 0.8% Red 34 Paste and 5.7% Red #7 LT Paste all available from Tevco, Inc. All pastes contain the lake form of the pigment, dispersed in a mixture of ethyl acetate, butyl acetate, isopropyl alcohol and nitrocellulose. The resulting mixture had a viscosity at 25 °C of 1,4 Pa.s (14 poise) at a shear rate of 2/sec shear rate and 1 Pa.s (10 poise) at 70/sec shear rate. The mixture was subdivided and one sample was maintained in a 50 °C oven and the other at ambient temperature with periodic checks made for settling. After 33 days, neither sample showed signs of settling.

### Example 6 (outside claim 1)

To 98 g of a UV-curable gel composition comprising 35% by weight of an aliphatic polyester based urethane multimethacrylate oligomer, 52% multifunctional acrylate monomers, 10% ethyl methacrylate and 2% photoinitiator was added 1.0 g of a polyurea urethane, (52% solution in N-methyl pyrrolidinone, available from Byk, Inc.). The resulting gel had a viscosity at 25 C of 22,76 Pa.s (227.6 poise) at 1/2sec shear rate and 2,372 Pa.s (23.72 poise) at 1/70sec shear rate. To this gel was added 10.0 g of a dispersion of 23.5% FD&C Red #6 aluminum lake pigment dispersed in a diacrylate monomer. The mixture was allowed to stand at ambient temperature and periodically checked for settling. After 6 months minimal syneresis was observed, no hard pack had formed. Upon shaking the material was easily resuspended and a homogeneous composition resulted.

### Example 6A (Comparative)

The above experiment was repeated without the addition of the polyurea urethane. Prior to pigment addition the gel composition had a viscosity at 25 C of 0,735 Pa.s (7.35 poise) at ½ sec shear and 0,64 Pa.s (6.4 poise) at 1/70 shear. The same pigment addition as in Example 1 was then performed and, after stirring, the composition was allowed to sit. After 24 hours syneresis was evident and after 120 hours a hard pack and the syneresis layer was >50% of the sample height.

### Example 7 (outside claim 1)

To 98.0 grams of the same UV curable gel of Example 6, 2.0 g of a castor oil derivative of a polyamide, was added. The resulting gel had a viscosity of 7,5 Pa.s (75 poise) at ½ sec shear rate and 1,858 Pa.s (18.58 poise) at 1/70 shear rate. To this gel was added 13 grams of a color concentrate consisting of 66.7% of a dispersion of 58.0% $TiO_2$ pigment dispersed in a diacrylate monomer and 32.3% of a dispersion of 23.5% FD&C Red #6 aluminum lake pigment dispersed in a diacrylate monomer. The mixture was allowed to stand at ambient temperature and periodically checked for settling. After 6 months minimal syneresis was observed, no hard pack had formed. Upon shaking, the material was easily resuspended and a homogeneous composition resulted without any deleterious application performance.

### Example 8 (outside claim 1)

To 97.0 g of a UV-curable gel comprising 58% by weight an aliphatic polyester based urethane multimethacrylate oligomer, 20% by weight hydroxyethylmethacrylate, 20 % by weight hydroxypropyl methacrylate and 2 % of a mixture of Irgacure 184 and Darocure TPO, both available from BASF Resins, as photoinititator was added 1.0 g of polyamide, and 2.0 g Texaphor Special dispersant, available through Cognis. To this was added 4.3 g of a color concentrate consisting of 17.8% of a dispersion of 58% $TiO_2$ pigment dispersed in a diacrylate monomer, 11.4% of a dispersion of 34.3% FD&C Yellow #5 aluminum lake pigment dispersed in a diacrylate monomer, 68.3% of a dispersion of 23.8% FD&C Red #7 aluminum lake pigment dispersed in a diacrylate monomer and 2.6% of a dispersion of 21.1% D&C Red #34 calcium lake pigment dispersed in a diacrylate monomer. The resulting composition had a viscosity at 25 C of 3,584 Pa.s (35.84 poise) at 1/2sec shear rate and 1,391 Pa.s (13.91 poise) at 1/70 shear rate. The mixture was allowed to stand at ambient temperature and periodically checked for settling. After 4 weeks, no settling had occurred.

### Example 8A (Comparative)

The above experiment was repeated without the addition of the polyamide (thixotropic additive) and Texaphor Special dispersant. The resulting colored composition had a viscosity at 25 C of 1, 111 Pa.s (11.11 poise) at 1/2sec shear and 1,018 Pa.s (10.18 poise) at 1/70 shear. After standing, initial syneresis was observed within 8 days, with a clear layer of approximately 15% of non-pigmented containing material observed at the top of the composition. Syneresis continues for an additional 10 days allowing for over 60% of the sample containing a clear layer.

### Example 8B (Comparative)

Formulation D of Patel, et al., U.S. Pat. 5,407,666, was prepared and compared to the gel composition of Ex. 4 representing the invention. Specifically, to 177.5g of ethyl methacrylate were added with stirring 37.6 g of a acrylic copolymer made up of ethyl methacrylate/methyl methacrylate copolymer, and 26.6 g of 2,2-dimethoxy-2-phenylacetoephenone. After dissolution of the polymer and initiator, 52.8 g of the solution was removed and to this was added 1.7g of fumed silica and 0.6 g of D&C Red 6 Ba Lake Pigment.

Coatings were placed on Colorpops™ available from Colorpops International and cured for 3 min with a Model EP-110V Lamp available from Keystone Research and Pharmaceutical, 616 Hollywood Avenue, Cherry Hill, New Jersey . Upon UV cure, 40.6 % by weight of the Patel, et al., composition was lost due to volitalization wherein only 0.4 % by weight of Ex. 4 representing the invention was lost due to volatilization.

The index and middle fingers of the right hand of a person were coated with the composition of Ex. 8, and the index and middle fingers of the left hand that person were coated with the composition of Ex. 8B. The coatings were cured for 3 minutes with a Model EP-110V UV Lamp available from Keystone Research and Pharmaceutical, 616 Hollywood Avenue, Cherry Hill, New Jersey. The coatings were worn for 2 days and then photos were taken of the two hands. The experiment was repeated, coating the index and middle fingers of the left hand of the same person with the composition of Ex. 8, and coating the index and middle fingers of the right hand with the composition of Ex. 8B. The coatings were worn for 2 days and then photos were taken of the two hands. The photos were assessed for wear by a panel of 9 people who ranked each coating from best to worst with 1 being the best and 8 the worst and also ranked them pairwise according to the finger on which the coating was applied . All coatings from Comparative Ex. 8B showed significant chipping after two days wear where only one coating from Example 8 showed significant chipping. Table 1 shows the average ranking by finger obtained. As can be seen all coatings using Example 8 outperformed those obtained from Comparative Example 8B.

**TABLE 1**

| Coating | Hand | Finger | Average Rank |
|---|---|---|---|
| Example 8 | Right | Index | 1 |
| Example 8 | Left | Index | 2.1 |
| Example 8 | Right | Middle | 2.9 |
| Example 8 | Left | Middle | 4.7 |
| Comparative Example 8B | Right | Index | 7.7 |
| Comparative Example 8B | Left | Index | 5.6 |
| Comparative Example 8B | Right | Middle | 5.8 |
| Comparative Example 8B | Left | Middle | 6.3 |

Data obtained from pairwise ratings of the same finger with two different coatings is shown in Table 2.

**Table 2- Number Of Raters Who Preferred Each Finger**

| Hand | Finger | Example 8 | Comparative Example 8B |
|---|---|---|---|
| Left | Index | 9 | 0 |
| Left | Middle | 8 | 1 |
| Right | Index | 9 | 0 |
| Right | Middle | 9 | 0 |

Once again the data shows superior wear for coatings from Example 8 even after only 2 days.

The present invention, therefore, is well adapted to carry out the objects and attain the ends and advantages mentioned,The depicted and described preferred embodiments of the invention are exemplary.

**Claims**

1. A composition comprising:

   (A) at least 35% by weight polyfunctional polyurethane (meth)acrylate oligomer;
   (B) at least 0.1% by weight pigment;
   (C) 0.5% to 10% by weight thixotropic additive selected from the group consisting of organically-modified clay, organically-modified mixed mineral clay, and a mixture thereof;
   (D) 0 to about 20% by weight non-reactive solvent;
   (E) 0 to no more than 20% by weight film-forming polymer;
   (F) 0.1 to 5% by weight photoinitiator;
   (G) 0 to 5% by weight dispersant, and
   (H) the balance to make 100% by weight of one or more mono-, di-tri-, and tetrafunctional acrylic or methacrylic monomers;

   wherein the ratio of oligomer (A) to film forming polymer (E) is greater than 2.5;
   and wherein the composition is a UV curable gel suitable for coating natural and artificial nails and forming a wear-resistant adornment, and
   wherein the composition has a low shear viscosity at 2/sec shear rate at 25°C of greater than 1.2 Pa.s and a high shear viscosity at 70/sec shear rate at 25°C of less than 2 Pa.s.

2. The composition of claim 1 having no film-forming polymer, (E), and no non-reactive solvent, (D).

3. The composition of claim 1 comprising a mixture of at least one colored pigment and $TiO_2$.

4. The composition of claim 1 wherein the reactive monomer (H) is selected from hydroxyethyl (meth)acrylate, hydroxypropyl (meth)acrylate, trimethylolpropane tri (meth)acrylate, and isobornyl (meth)acrylate.

5. The composition of claim 1 wherein the oligomer (A) is an aliphatic polyester based urethane diacrylate oligomer.

6. A method comprising applying a composition according to claim 1 onto human nails or artificial nails and curing under UV radiation.

7. An artificial, pigment containing nail coating prepared by curing under actinic radiation a composition according to claim 1.

8. The composition according to claim 1 wherein the monomer is mono- or difunctional.

**Patentansprüche**

1. Zusammensetzung, umfassend:

   (A) mindestens 35 Gewichts% polyfunktionelles Polyurethan(meth)acrylatoligomer;
   (B) mindestens 0,1 Gewichts% Pigment;
   (C) 0,5 Gewichts% bis 10 Gewichts% thixotropen Zusatzstoff, ausgewählt aus der Gruppe bestehend aus organisch modifiziertem Ton, organisch modifiziertem gemischtem Mineralton und einer Mischung davon;
   (D) 0 bis etwa 20 Gewichts% reaktionsunfähiges Lösungsmittel;
   (E) 0 bis höchstens 20 Gewichts% filmbildendes Polymer;
   (F) 0,1 bis 5 Gewichts% Fotoinitiator;
   (G) 0 bis 5 Gewichts% Dispersionsmittel und
   (H) eines oder mehrere von mono-, bi-, tri- und tetrafunktionellen Acryl- oder Methacrylmonomeren als Rest auf 100 Gewichts%;

   wobei das Verhältnis von Oligomer (A) zu filmbildendem Polymer (E) größer als 2,5 ist;
   und wobei die Zusammensetzung ein UV-härtbares Gel umfasst, das zum Beschichten natürlicher und künstlicher Fingernägel und Bilden einer verschleißfesten Verzierung geeignet ist, und
   wobei die Zusammensetzung eine niedrige Scherviskosität mit einer Scherrate von 2/sec bei 25 °C von mehr als 1,2 Pa·s und eine hohe Scherviskosität mit einer Scherrate von 70/sec bei 25 °C von weniger als 2 Pa·s aufweist.

2. Zusammensetzung nach Anspruch 1 ohne filmbildendes Polymer (E) und ohne reaktionsunfähiges Lösungsmittel (D).

3. Zusammensetzung nach Anspruch 1, umfassend ein Gemisch aus mindestens einem Buntpigment und $TiO_2$.

4. Zusammensetzung nach Anspruch 1, wobei das reaktionsfähige Monomer (H) aus Hydroxyethyl(meth)acrylat, Hydroxypropyl(meth)acrylat, Trimethylolpropantri(meth)acrylat und Isobornyl(meth)acrylat ausgewählt ist.

5. Zusammensetzung nach Anspruch 1, wobei das Oligomer (A) ein Urethandiacrylatoligomer auf der Basis eines aliphatischen Polyesters ist.

6. Verfahren, umfassend ein Auftragen einer Zusammensetzung nach Anspruch 1 auf menschliche Fingernägel oder künstliche Fingernägel und Aushärten unter UV-Strahlung.

7. Künstliches Pigment, das eine Fingernagelbeschichtung enthält, die durch Aushärten unter aktinischer Bestrahlung einer Zusammensetzung nach Anspruch 1 hergestellt ist.

8. Zusammensetzung nach Anspruch 1, wobei das Monomer mono- oder bifunktionell ist.

**Revendications**

1. Composition comprenant :

(A) au moins 35 % en poids d'un oligomère de poly(méth)acrylate d'uréthane polyfonctionnel ;

(B) au moins 0,1 % en poids d'un pigment ;

(C) 0,5 % à 10 % en poids d'un additif thixotropique choisi dans le groupe constitué par une argile à modification organique, une argile minérale mixte à modification organique, et un mélange de celles-ci ;

(D) 0 à environ 20 % en poids d'un solvant non réactif ;

(E) 0 à au plus 20 % en poids d'un polymère filmogène ;

(F) 0,1 à 5 % en poids d'un photoamorceur ;

(G) 0 à 5 % en poids d'un dispersant ; et

(H) le reste à 100 % en poids d'un ou plusieurs monomères acryliques ou méthacryliques mono-, di-, tri- et tétra-fonctionnels ;

dans laquelle le rapport de l'oligomère (A) au polymère filmogène (E) est supérieur à 2,5 ;

et laquelle composition est un gel durcissable aux UV convenant pour revêtir des ongles naturels et artificiels et former un ornement résistant à l'usure,

et laquelle composition a une viscosité sous faible cisaillement à une vitesse de cisaillement de 2/s à 25°C supérieure à 1,2 Pa.s et une viscosité sous fort cisaillement à une vitesse de cisaillement de 70/s à 25°C inférieure à 2 Pa.s.

2. Composition selon la revendication 1, n'ayant pas de polymère filmogène (E) et pas de solvant non réactif (D).

3. Composition selon la revendication 1, comprenant un mélange d'au moins un pigment coloré et de $TiO_2$.

4. Composition selon la revendication 1, dans laquelle le monomère réactif (H) est choisi parmi le (méth)acrylate d'hydroxyéthyle, le (méth)acrylate d'hydroxypropyle, le tri(méth)acrylate de triméthylolpropane et le (méth)acrylate d'isobornyle.

5. Composition selon la revendication 1, dans laquelle l'oligomère (A) est un oligomère de diacrylate d'uréthane à base de polyester aliphatique.

6. Procédé comprenant l'application d'une composition selon la revendication 1 sur des ongles humains ou des ongles artificiels et le durcissement sous un rayonnement UV.

7. Vernis à ongles contenant un pigment artificiel préparé par durcissement sous un rayonnement actinique d'une composition selon la revendication 1.

8. Composition selon la revendication 1, dans laquelle le monomère est mono- ou di-fonctionnel.

**EP 2 525 773 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 80425310
- US 79895310
- US 4682612 A
- US 6244274 B
- US 6555096 B
- US 4222908 A
- US 5985951 A, Cook
- US 6051242 A
- US 5407666 A, Patel